# EUROPEAN PATENT APPLICATION

(11) **EP 2 608 091 A2**
(43) Date of publication of application: **26.06.2013**
(21) Application number: 12191415.4
(22) Date of filing: 06.11.2012
(51) Int. Cl.: G06F 19/00

(54) **Diagnostic factor set determination apparatus and method**

(30) Priority: 21.12.2011 KR 20110139503
(71) Applicant: Samsung Electronics Co., Ltd, Gyeonggi-do 443-742 (KR)
(72) Inventor: Kim, Ha-Young, 103-3002 Gyeonggi-do (KR); Woo, Kyoung-Gu, 103-1602 Seoul (KR); Lee, Ji-Hyun, A-3009 Gyeonggi-do (KR)
(74) Representative: Grünecker, Kinkeldey, Stockmair & Schwanhäusser

(57) **Abstract**

A diagnostic factor set determination apparatus and method is provided. The diagnostic factor set determination apparatus includes a personal examination data acquiring unit configured to acquire personal examination data including a plurality of diagnostic factors, a disease model selecting unit configured to select a disease model that includes one or more of the plurality of diagnostic factors, and a diagnostic factor processing unit configured to determine a diagnostic factor set according to a sum of disease weights of a first group of the plurality of diagnostic factors that is not in the selected disease model.

## Description

### BACKGROUND

### 1. Field

The following description relates to a data acquisition technology for disease diagnosis, and, for example, to a diagnostic factor set determination apparatus and method.

### 2. Description of Related Art

Diagnostic factors included in personal examination data may vary depending on the situation in which a patient is examined, the hospital in which the patient examination is conducted, and the area or country in which the patient examination is conducted. By using a previously created disease model based on personal examination data from various examinations having different diagnostic factors to determine diagnostic factors for a target disease, diagnostic factors not included in the disease model may be processed according to a variety of methods to predict the occurrence of disease or assess the severity of ongoing disease.

In one method, disease diagnosis is performed based on only the diagnostic factors included in the disease model. Diagnostic factors not included in the disease model are ignored in the disease diagnosis. In another method, disease diagnosis is performed by classifying diagnostic factors not included in the disease model into a particular category. In yet another method, disease diagnosis is performed by ignoring diagnostic factors that are not included in the disease model and reweighting personal examination data. In still another method, disease diagnosis is performed by substituting diagnostic factors not included in the disease model with valid diagnostic factors. However, the methods that ignore the diagnostic factors not included in the disease model or substitute these excluded diagnostic factors with valid diagnostic factors may not ensure accurate prediction of the occurrence of disease or accurate assessment of the severity of disease.

### SUMMARY

In one general aspect, there is provided a diagnostic factor set determination apparatus, including a personal examination data acquiring unit configured to acquire personal examination data including a plurality of diagnostic factors, a disease model selecting unit configured to select a disease model that includes one or more of the plurality of diagnostic factors, and a diagnostic factor processing unit configured to determine a diagnostic factor set according to a sum of disease weights of a first group of the plurality of diagnostic factors that is not in the selected disease model. This improves the accuracy of a prediction of the occurrence of disease or of an assessment of the severity of disease. For example, the diagnostic factors may include one or more of blood pressure, cholesterol level, body weight, and the like. A disease weight of the diagnostic factor may generally refers to a degree of influence of a diagnostic factor in a disease model.

The general aspect of the apparatus may further provide that the diagnostic factor processing unit includes a diagnostic factor determination unit configured to determine diagnostic factors of a new disease model from the plurality of diagnostic factors if the sum is determined to be equal to or greater than a first threshold value. This has the advantage that a new disease model can be easily found.

The general aspect of the apparatus may further provide that the diagnostic factor processing unit further includes a comparison unit configured to conduct a comparison of the first threshold value and the sum and provide a result of the comparison to the diagnostic factor determination unit to determine the diagnostic factors of the new disease model. This provides a simple implementation of the determination if the sum is equal to or greater than the first threshold value.

The general aspect of the apparatus may further provide that, if the comparison result indicates that the sum is less than the first threshold value, the diagnostic factor determination unit determines diagnostic factors included in both the personal examination data and the selected disease model as the diagnostic factor set for disease diagnosis. Accordingly, it can be determined that the selected disease model fits to the personal examination data.

The general aspect of the apparatus may further provide that the diagnostic factor determination unit is further configured to include a second group of the plurality of diagnostic factors in the diagnostic factors of the new disease model, the second group being in the acquired personal examination data and the selected disease model. This provides further data to the new disease model.

The general aspect of the apparatus may further provide that the diagnostic factor determination unit is further configured to identify correlated diagnostic factors between diagnostic factors of the first group and diagnostic factors of the selected disease model that are not in the acquired personal examination data, and include the correlated diagnostic factors in the diagnostic factors of the new disease model. This increases the reliability of the new disease model.

The general aspect of the apparatus may further provide that, if the correlated diagnostic factors are not identified, the diagnostic factor determination unit calculates relations between a target disease and each of the diagnostic factors of the first group, and includes a third group of diagnostic factors of the first group in the diagnostic factors of the new disease model, the diagnostic factors of the third group respectively having calculated relations that are greater than the calculated relations of any other of the diagnostic factors of the first group. These relations may correspond to the disease weights.

The general aspect of the apparatus may further provide that, if the sum is determined to be less than a second threshold value, the diagnostic factor determining unit determines one diagnostic factor of the diagnostic factors of the first group having one of the calculated relations that is greater than the calculated relations of any other of the diagnostic factors of the first group, and includes the determined one diagnostic factor in the diagnostic factors of the new disease model. If the sum is determined to be equal to or greater than the second threshold value, the diagnostic factor determination unit determines two or more diagnostic factors of the diagnostic factors of the first group respectively having calculated relations that are greater than the calculated relations of any other of the diagnostic factors of the first group of the plurality of diagnostic factors, and includes the determined two or more diagnostic factors in the diagnostic factors of the new disease model. This allows selective determination of diagnostic factors relevant in a particular disease model.

The general aspect of the apparatus may further provide that the diagnostic factor processing unit further includes a disease model creation unit configured to create the new disease model to include the determined diagnostic factors.

The general aspect of the apparatus may further provide that the diagnostic factor determination unit is further configured to classify the determined diagnostic factors as the diagnostic factor set. This provides a convenient way to for example mark the determined diagnostic factors as elements of the diagnostic factor set.

The general aspect of the apparatus may further provide that the disease model creation unit is further configured to compute a first plurality of patients having a disease including the determined diagnostic factors by searching a patient database (DB). If the computed first plurality of patients is equal to or greater than a third threshold value, the disease model creation unit creates the new disease model to include the determined diagnostic factors. If the computed first plurality of patients is less than the third threshold value, the disease model creation unit computes a second plurality of patients having a target disease that includes the determined diagnostic factors except for one of the determined diagnostic factors having a relation with the target disease that is less than relations of any other of the determined diagnostic factors with the target disease. This further increases the amount of input data for the creation of new disease models.

The general aspect of the apparatus may further provide that the diagnostic factor processing unit further includes a disease model registration unit configured to register the created new disease model in a disease model database (DB). This provides for future reference of the new disease model.

The general aspect of the apparatus may further provide that the disease model DB stores disease models for a plurality of diseases, the plurality of diseases including the target disease. The disease model selecting unit is further configured to find disease models corresponding to the target disease by searching the disease model DB. The disease model selecting unit is further configured to select one of the disease models corresponding to the target disease that includes the one or more of the plurality of diagnostic factors. This provide for a fast implementation to find disease models corresponding to a particular disease.

The general aspect of the apparatus may further provide that the disease model registration unit is further configured to search a patient model DB for patients having a target disease comprising the determined diagnostic factors. The disease model registration unit is further configured to classify patients found from the search of the patient model DB into a group of patients having the target disease and a group of patients not having the target disease. If diagnosis accuracy is measured with respect to the group of patients not having the target disease and the diagnosis accuracy is equal to or greater than a fourth threshold value, the disease model registration unit registers the created new disease model in the disease model DB. Accordingly, a specific relevance of the determined diagnostic factors can be set.

The general aspect of the apparatus may further provide that the disease model selecting unit is further configured to select the disease model from a plurality of disease models including one or more of the plurality of diagnostic factors, the selected disease model having a diagnosis accuracy that is greater than diagnosis accuracies of any other disease model of the plurality of disease models. Accordingly, a disease model with probably the highest relevance may be found.

The general aspect of the apparatus may further provide that the selected disease model is one of a plurality of disease models. The selected disease model includes a greater number of the plurality of diagnostic factors than any other disease model of the plurality of disease models. This is a simple condition for selecting the disease model that probably is the most relevant in view of the target disease.

In another general aspect, there is provided a diagnostic factor set determination method, including acquiring personal examination data including a plurality of diagnostic factors, selecting a disease model that including one or more of the plurality of diagnostic factors of the acquired personal examination data; and determining a diagnostic factor set according to a sum of disease weights of a first group of the plurality of diagnostic factors that is not in the selected disease model. Here and in the following, the advantages of the method features correspond to the respective advantages of the corresponding apparatus features.

The general aspect of the method may further provide conducting a comparison of the first threshold value and the sum, and, if the comparison determines that the sum is equal to or greater than the first threshold value, determining diagnostic factors of a new disease model from the plurality of diagnostic factors.

The general aspect of the method may further provide, if a result of the comparison indicates that the sum is less than the first threshold value, determining diagnostic factors included in both the personal examination data and the selected disease model as the diagnostic factor set for disease diagnosis.

The general aspect of the method may further provide creating the new disease model to include the determined diagnostic factors, registering the created new disease model in a disease model database (DB), and classifying the determined diagnostic factors as the diagnostic factor set.

The general aspect of the method may further provide that the determining of the diagnostic factors includes including a second group of the plurality of diagnostic factors in the diagnostic factors of the new disease model, the second group being in the acquired personal examination data and the selected disease model.

The general aspect of the method may further provide that the determining of the diagnostic factors further includes identifying correlated diagnostic factors and including the correlated diagnostic factors in the diagnostic factors of the new disease model, the correlated diagnostic factors being identified between diagnostic factors of the first group and diagnostic factors of the selected disease model that are not in the acquired personal examination data.

The general aspect of the method may further provide that the determining of the diagnostic factors further includes, if the correlated diagnostic factors are not identified, calculating relations between a target disease and each of the diagnostic factors of the first group, and including a third group of diagnostic factors of the first group in the diagnostic factors of the new disease model, the diagnostic factors of the third group respectively having calculated relations that are greater than the calculated relations of any other of the diagnostic factors of the first group.

The general aspect of the method may further provide that the selected disease model is one of a plurality of disease models. The selected disease model includes a greater number of the plurality of diagnostic factors than any other disease model of the plurality of disease models.

The provisions of the general aspect of the invention may be implemented separately or in combination with each other.

Other features and aspects may be apparent from the following detailed description, the drawings, and the claims.

### BRIEF DESCRIPTION OF THE DRAWINGS

FIG. 1 is a diagram illustrating an example of a diagnostic factor set determination apparatus.

FIG. 2 is a diagram illustrating an example of how to select a disease model using the diagnostic factor set determination apparatus.

FIG. 3 is a diagram illustrating an example of a diagnostic factor set determined using the diagnostic factor set determination apparatus.

FIG. 4 is a diagram illustrating another example of a diagnostic factor set determined using the diagnostic factor set determination apparatus..

FIG. 5 is a diagram illustrating yet another example of a diagnostic factor set determined using the diagnostic factor set determination apparatus.

FIG. 6 is a flowchart illustrating an example of a diagnostic factor set determination method.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. In addition, descriptions of well-known functions and constructions may be omitted for increased clarity and conciseness.

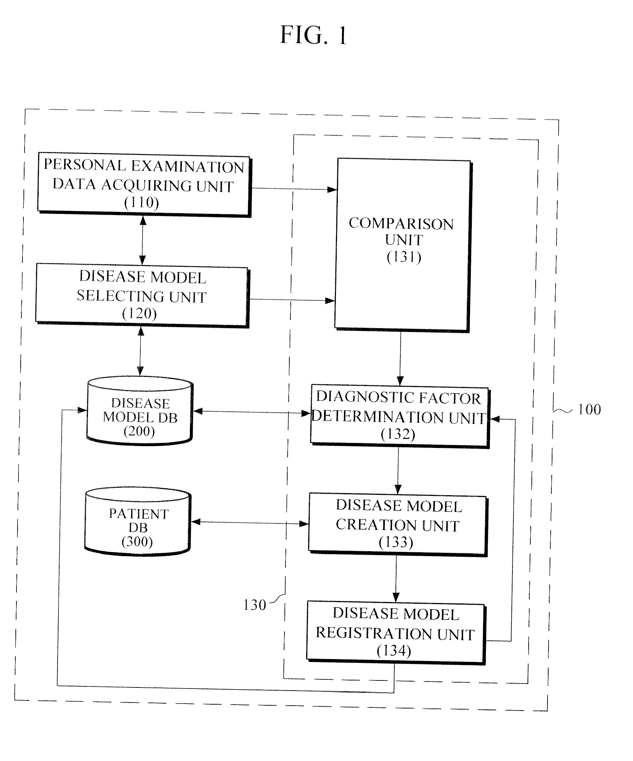

FIG. 1 is a diagram illustrating an example of a diagnostic factor set determination apparatus 100. Referring to FIG. 1, the apparatus 100 includes a personal examination data acquiring unit 110, a disease model selecting unit 120, and a diagnostic factor processing unit 130.

The personal examination data acquiring unit 110 may acquire personal examination data including one or more diagnostic factors. For example, the diagnostic factors may include blood pressure, cholesterol level, body weight, and the like, which are included for diagnosing disease. The personal examination data acquiring unit 110 may obtain personal examination data from a memory (not shown) that stores a variety of personal examination data including one or more diagnostic factors. The personal examination data acquiring unit 110 may also acquire personal examination data including one or more diagnostic factors from other devices (not shown) through wired/wireless communication. The personal examination data acquiring unit 110 may include a user interface (UI) to allow a user to input personal examination data including one or more diagnostic factors through the UI.

The disease model selecting unit 120 may select a disease model that includes one or more diagnostic factors included in the personal examination data acquired by the personal examination data acquiring unit 110. For example, the disease model selecting unit 120 may select a disease model having the greatest amount of diagnostic factors included in the personal examination data acquired by the personal examination data acquiring unit 110.

The disease model selecting unit 120 may search for disease models corresponding to a target disease from a disease model database (DB) 200 that stores disease models for each disease. The disease model selecting unit 120 may select a disease model that includes one or more diagnostic factors included in the personal examination data. Target disease information may be input or selected by a user. Where each of a plurality of disease models includes one or more diagnostic factors included in the personal examination data, the disease model selecting unit 120 may select a disease model having greater diagnosis accuracy than any of the other disease models. The diagnosis accuracy is a value that is previously set for each of the disease models. The diagnosis accuracy value serves to indicate an accuracy of each disease model in a diagnosis of a target disease.

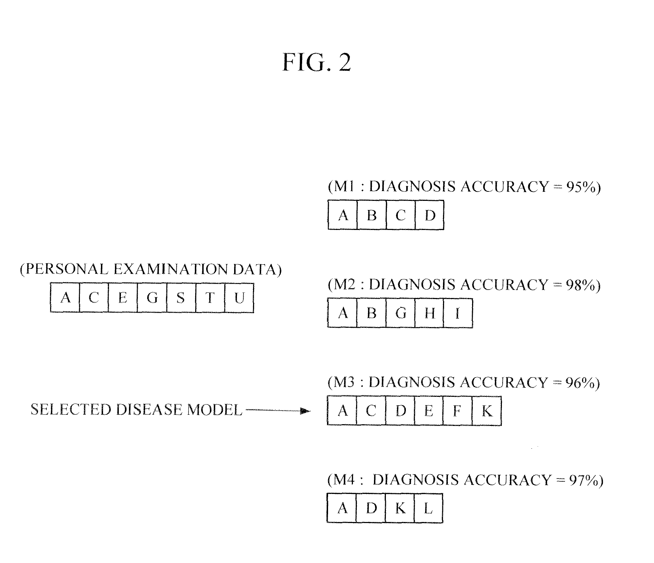

FIG. 2 is a diagram illustrating an example of how to select a disease model using the diagnostic factor set determination apparatus 100. In FIG. 2, a plurality of previously stored disease models for a target disease include disease model M1, which includes diagnostic factors A, B, C, and D and has a diagnosis accuracy of 95%, disease model M2, which includes diagnostic factors A, B, G, H, and I and has a diagnosis accuracy of 98%, disease model M3, which includes diagnostic factors A, C, D, E, F, and K and has a diagnosis accuracy of 96%, and disease model M4, which includes diagnostic factors A, D, K, and L and has a diagnosis accuracy of 97%.

When personal examination data acquired by the personal examination data acquiring unit 110 includes diagnostic factors A, C, E, G, S, T, and U, the disease model selecting unit 120 selects disease model M3, which has the greatest amount of diagnostic factors (A, C, and E) included in the personal examination data acquired by the personal examination data acquiring unit 110.

The diagnostic factor processing unit 130 may determine a diagnostic factor set to diagnose a disease based on a sum of disease weights of the respective diagnostic factors that belong to the personal examination data and are not included in the selected disease model. A disease weight of the diagnostic factor refers to an influence of a diagnostic factor on a target disease. The disease weight is previously set and stored for each of the diseases.

As shown in FIG. 2, in response to the disease model selecting unit 120 selecting disease model M3, which includes diagnostic factors, A, C, D, E, F, and K, the diagnostic factor processing unit 130 may determine a diagnostic factor set based on the sum of disease weights of diagnostic factors G, S, T, and U, which belong to the personal examination data but are not included in the selected disease model M3.

According to the teachings above, in contrast to a conventional diagnostic factor determining method in which diagnostic factors not included in a disease model are ignored or substituted by valid diagnostic factors, there is provided a diagnostic factor set determination apparatus that may determine the diagnostic factor set based on the sum of disease weights of diagnostic factors that belong to the personal examination data but are not included in a disease model, thereby fully reflecting the diagnostic factors provided in the personal examination data in a disease diagnosis. As a result, disease diagnosis may be made more accurately.

In another example, the diagnostic factor processing unit 130 includes a comparison unit 131 and a diagnostic factor determination unit 132. The comparison unit 131 may compare a first threshold with the sum of the disease weights of diagnostic factors that belong to the personal examination data but are not included in the disease model.

As shown in FIG. 2, when disease model M3, which includes diagnostic factors A, C, D, E, F, and K, is selected by the disease model selecting unit 120, the comparison unit 131 may compare the first threshold value with the sum of the disease weights of diagnostic factors G, S, T, and U, which belong to the personal examination data but are not included in the selected disease model M3. Based on a result of a comparison by the comparison unit 131, if the sum of the disease weights of the diagnostic factors is equal to or greater than the first threshold value, the diagnostic factor determination unit 132 may determine diagnostic factors G, S, T, and U to be diagnostic factors of a new disease model created with respect to the personal examination data.

The diagnostic factor determination unit 132 may determine the diagnostic factors that are included in both the personal examination data and the selected disease model to be diagnostic factors of the new disease model. As shown in FIG. 2, where the disease model selecting unit 120 selects disease model M3, which includes diagnostic factors A, C, D, E, F, and K, the diagnostic factor determination unit 132 may determine diagnostic factors A, C, and E, which are included in both the disease model M3 and the personal examination data, to be diagnostic factors of the new disease model.

In addition, the diagnostic factor determination unit 132 may analyze a correlation between the diagnostic factors that are included in the personal examination data but are not included in the selected disease model and the diagnostic factors that are included in the selected disease model but are not included in the personal examination data. The diagnosis factor determination unit 132 may determine to further include the diagnostic factors that are included in the personal examination data and are correlated largely with the diagnostic factors included in the selected disease model in the newly created disease model. The correlation is previously set and stored information indicating how the diagnostic factors that are included in the personal examination data but are not included in the selected disease model are related to the diagnostic factors that are included in the selected disease model but are not included in the personal examination data.

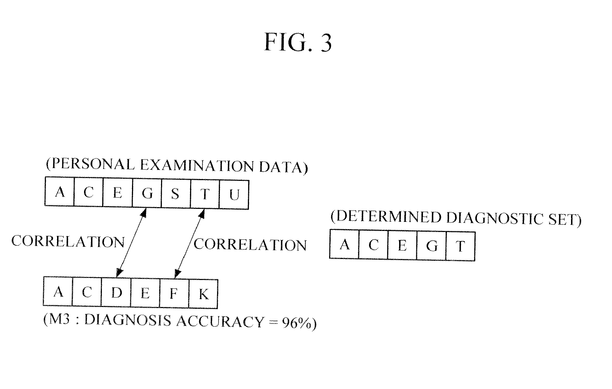

FIG. 3 is a diagram illustrating an example of a determined diagnostic factor set using the diagnostic factor set determination apparatus 100. As shown in FIG. 2, in a case in which the disease model selecting unit 120 selects disease model M3 and the personal examination data includes diagnostic factors A, C, E, G, S, T, and U, the diagnostic factor determination unit 132 may analyze the correlation between the diagnostic factors G, S, T, and U, which are in the personal examination data but not in the selected disease model, and the diagnostic factors D, F, and K, which are in the selected disease model but not in the personal examination data. If a result of a correlation analysis shows that the diagnostic factor D included in the selected disease model is correlated with the diagnostic factor G included in the personal examination data and the diagnostic factor F included in the selected disease model is correlated with the diagnostic factor T, the diagnostic factor determination unit 132 may determine diagnostic factors G and T as diagnostic factors of a new disease model, as shown in FIG. 3.

If a result of a correlation analysis indicates that there is no correlation between the above-referenced diagnostic factors, the diagnostic factor determination unit 132 may calculate a relation between each diagnostic factor in the personal examination data and the target disease, and determine a diagnostic factor of a new disease model to be the diagnostic factor having a relation with the target disease that is greater than a relation of any of the other diagnostic factors. The relations between each of the diagnostic factors in the personal examination data and the target disease are previously set and stored to serve as an indication of an impact of each of the diagnostic factors on the target disease.

In the above example, if a result of a correlation analysis shows that there is no correlation between the diagnostic factors G, S, T, and U, which are in the personal examination data but are not in the selected disease model, and the diagnostic factors D, F, and K, which are in the selected disease model but not in the personal examination data, the diagnostic factor determination unit 132 may calculate a relation between each of the diagnostic factors G, S, T, and U, and further determine a diagnostic factor of a new disease model to be the diagnostic factor having a relation with the target disease that is greater than a relation of any of the other diagnostic factors.

The diagnostic factor determination unit 132 compares a second threshold value with the sum of disease weights of the diagnostic factors that are in the personal examination data but not in the selected disease model. If the sum of disease weights of the diagnostic factors is less than the second threshold value, the diagnostic factor determination unit 132 may determine a diagnostic factor of a new disease model to be the diagnostic factor having a relation with the target disease that is greater than a relation of any of the other diagnostic factors with the target disease.

In other words, if the sum of disease weights of the diagnostic factors in the personal examination data is less than the second threshold value, the diagnostic factors in the personal examination data may be determined to be less related with the target disease than the diagnostic factor having the above-referenced relation with a target disease. Thus, only the diagnostic factor having a relation with the target disease that is greater than a relation of any of the other diagnostic factors with the target disease may be determined as a diagnostic factor of a new disease model.

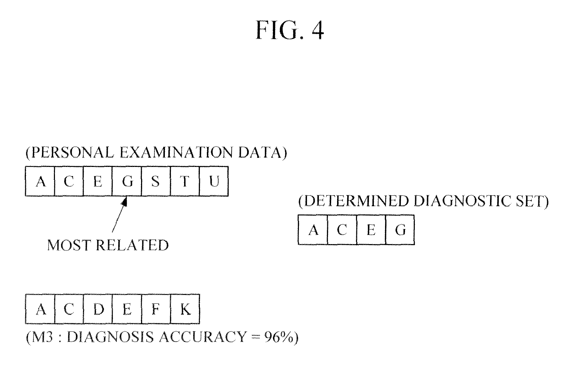

FIG. 4 is a diagram illustrating another example of a diagnostic factor set determined using the diagnostic factor set determination apparatus. For example, if the relations of each of the diagnostic factors G, S, T, and U, which are in the personal examination data but not in the selected disease model, with the target disease are obtained as G > S > T > U, and the sum of the disease weights of the diagnostic factors G, S, T, and U is less than the second threshold value, the diagnostic factor determination unit 132 may determine the diagnostic factor G to be provided for a new disease model, as shown in FIG. 4, since the diagnostic factor G has a relation with the target disease that is greater than a relation of any of the other diagnostic factors with the target disease.

If a result of comparison with the second threshold value shows that the sum of the disease weights of the diagnostic factors that are in the personal examination data but not in the selected disease model is equal to or greater than the second threshold value, the diagnostic factor determination unit 132 may determine to provide two or more diagnostic factors as having relations with the target disease that are greater than a relation of any of the other diagnostic factors with the target disease to create a new disease model.

If the sum of disease weights of the diagnostic factors in the personal examination data is equal to or greater than the second threshold value, the two or more diagnostic factors may have greater relations with the target disease than any other of the diagnostic factors in the personal examination data. Thus, the two or more diagnostic factors may be provided for a new disease model.

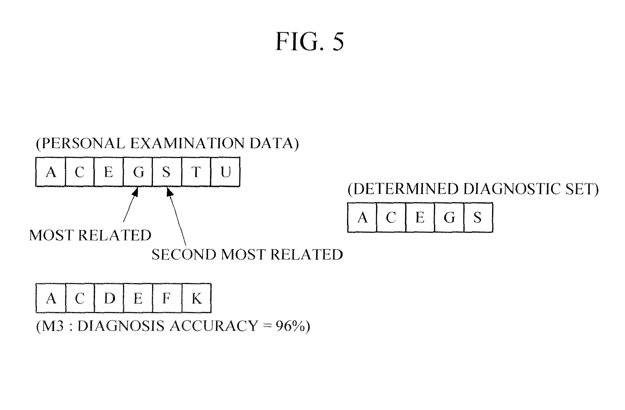

FIG. 5 is a diagram illustrating yet another example of a diagnostic factor set determined using the diagnostic factor set determination apparatus. For example, if the relations of each of the diagnostic factors G, S, T, and U, which are in the personal examination data but not in the selected disease model, with the target disease are obtained as G > S > T > U, and the sum of the disease weights of the diagnostic factors G, S, T, and U is equal to or greater than the second threshold value, the diagnostic factor determination unit 132 may determine two diagnostic factors G and S to have relations with the target disease that are greater than relations between the target disease and any other of the diagnostic factors that are in the personal examination data but not in the selected disease model. As a result, the two diagnostic factors G and S may be provided for a new disease model, as shown in FIG. 5.

In another example, the diagnostic factor processing unit 130 includes a disease model creation unit 133. The disease model creation unit 133 may create a new disease model that includes the diagnostic factors determined by the diagnostic factor determination unit 132.

The disease model creation unit 133 may search a patient DB 300 to compute a number of patients who have a target disease including the diagnostic factors determined by the diagnostic factor determination unit 132. If the computed number of patients is equal to or greater than a third threshold value, the disease model creation unit 133 may create a new disease model that includes the determined diagnostic factors.

A sufficient number of patients who have the target disease including the diagnostic factors determined by the diagnostic factor determination unit 132 should be identified in order for the creation of new disease model. The third threshold value corresponds to an identification of a sufficient number of patients. For example, if there are a sufficient number of patients who have the target disease, which would be indicated by the number of patients being equal to or greater than the third threshold value, the disease model creation unit 133 may create a new disease model that includes the diagnostic factors determined by the diagnostic factor determination unit 132.

On the contrary, if the number of patients is less than the third threshold value, the disease model creation unit 133 may compute the number of patients who have a disease that includes the determined diagnostic factors, while excluding the diagnostic factor of the determined diagnostic factors having a relation with the target disease that is less than relations of any other of the determined diagnostic factors with the target disease. For example, the disease model creation unit 133 may use the third threshold value to detect whether there are a sufficient number of patients who have the target disease including the diagnostic factors determined by the diagnostic factor determination unit 132, so that a new disease model can be created. If the number of patients is not sufficient to create a new disease model, the disease model creation unit 133 may detect whether the number of patients who have a disease that includes the determined diagnostic factors and excludes the diagnostic factor of the determined diagnostic factors having a relation with the target disease that is less than relations of any other of the determined diagnostic factors with the target disease is sufficient to create a new disease model. If there are a sufficient number of patients who have the disease that includes the determined diagnostic factors and excludes the diagnostic factor of the determined diagnostic factors having a relation with the target disease that is less than relations of any other of the determined diagnostic factors with the target disease, the disease model creation unit 133 may dynamically create a new disease model.

In another example, the diagnostic factor processing unit 130 includes a disease model registration unit 134. The disease model registration unit 134 may register the new disease model created by the disease model creation unit 133 in a disease model DB 200. By the disease model registration unit 134 registering and storing the new disease model in the disease model DB 200, the new disease model may be used for future determination of an optimal diagnostic model set.

The disease model registration unit 134 may search the patient DB 300 for the patients who show the diagnostic factors determined by the diagnostic factor determination unit 132, and classify those patients who are found to show the diagnostic factors into a group of patients having the target disease and a group of patients not having the target disease. In addition, the disease model registration unit 134 may evaluate the accuracy of the new disease model with respect to the group of patients not having the target disease. If the measured accuracy is equal to or greater than a fourth threshold value, the disease model registration unit 134 may register the new disease model in the disease model DB 200.

That is, if the number of patients who have the target disease including the diagnostic factors determined by the diagnostic factor determination unit 132 is sufficient to create a new disease model, the disease model registration unit 134 may evaluate the accuracy of the new disease mode in diagnosing the target disease. If an evaluation result shows that the accuracy of the new disease model is reliable, the created new disease model is registered in the disease model DB 200.

In another example, the diagnostic factor determination unit 132 may make a diagnostic factor set with the diagnostic factor included in the new disease model registered by the disease model registration unit 134.

In addition, when a comparison result of the comparison unit 131 indicates that the sum of disease weights of diagnostic factors is less than the first threshold value, the diagnostic factors may not affect the disease diagnosis. As a result, the diagnostic factor determination unit 132 may make a diagnostic factor set with the diagnostic factors belonging to both the personal examination data and the disease model selected by the disease model selecting unit 120.

If a comparison result of the comparison unit 131 indicates that the sum of disease weights of the diagnostic factors that are in the personal examination data but not in the selected disease model is equal to or greater than the first threshold value, the diagnostic factors may affect the disease diagnosis.

That is, if it is indicated that the diagnostic factors that are in the personal examination data but not in the selected disease model do not impact the disease diagnosis, the diagnostic factors that are included in both the personal examination data and the disease model selected by the disease model selection unit 120 are determined to be provided as a diagnostic factor set.

A disease diagnostic apparatus (not shown) analyzes the diagnostic factor set, which is determined by the diagnostic factor determination unit 132, to diagnose a disease, thereby predicting the occurrence of disease or assessing the severity of ongoing disease.

In this example, the disease diagnostic apparatus may be implemented to be physically or logically integrated with the diagnostic factor set determination apparatus 100, or implemented to be physically or logically separate from the diagnostic factor set determination apparatus 100.

The diagnostic factor set determination apparatus 100 may allow the diagnostic factor set to be made according to the sum of disease weights of the diagnostic factors that are in the personal examination data but not in the selected disease model. As a result, accurate and reliable disease diagnosis may be possible.

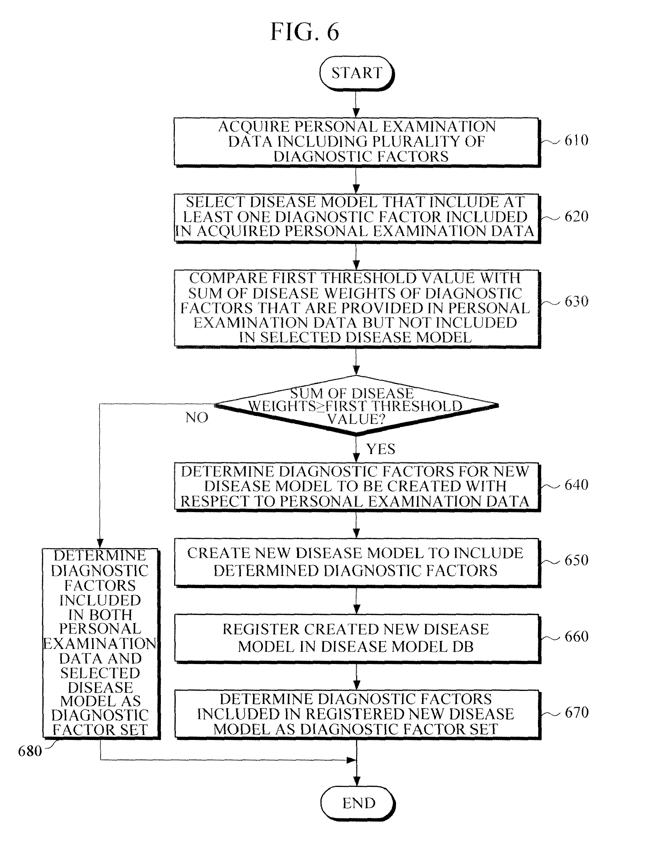

FIG. 6 is a flowchart illustrating an example of a diagnostic factor set determination method. As illustrated in FIG. 6, personal examination data, including a plurality of diagnostic factors, is acquired (610). For example, the diagnostic factors may be information, such as blood pressure, cholesterol level, and body weight, that may be required for diagnosing a disease. In addition, the personal examination data including the diagnostic factors may be acquired from a memory (not shown), from another device through wired or wireless communications, or through a user interface allowing the input of the personal examination data.

A disease model that includes one or more diagnostic factors included in the acquired personal examination data is selected (620). For example, a disease model that has the greatest number of diagnostic factors included in the acquired personal examination data may be selected.

In another example, a disease model that includes one or more diagnostic factors provided in the personal examination data may be selected from a plurality of disease models found in a disease model DB corresponding to a target disease. The disease model DB may store disease models for each disease. If there are more than two disease models that include one or more diagnostic factors provided in the personal examination data, a disease model having higher diagnosis accuracy may be selected.

A first threshold value is compared (630) with the sum of disease weights of the diagnostic factors in the personal examination data but not in the selected disease model. If a comparison result illustrates the sum of disease weights of the diagnostic factors to be equal to or greater than the first threshold value, diagnostic factors are determined (640) for a new disease model to be created with respect to the personal examination data. The diagnostic factors that are included in both the personal examination data and the selected disease model may be determined as diagnostic factors of the new disease model.

In addition, correlations between the diagnostic factors that are in the personal examination data but not in the selected disease model and the diagnostic factors belonging to the selected disease model but not included in the personal examination data are analyzed. The diagnostic factors provided in the personal examination data that are determined through analysis to be correlated with the diagnostic factor belonging to the selected disease model may be provided to the new disease model.

On the contrary, if there are no correlations between the diagnostic factors in the personal examination data but not in the selected disease model and the diagnostic factors in the selected disease model but not in the personal examination data, relations between the target disease and each of the diagnostic factors in the personal examination data but not in the selected disease model may be calculated. As a result, the diagnostic factor being more related with the target disease may be determined to be provided to the new disease model.

The sum of disease weights of the diagnostic factors in the personal examination data but not in the selected disease model may be compared with a second threshold value. If the sum of disease weights is less than the second threshold value, the diagnostic factor being most related with the target disease may be determined to be provided to the new disease model.

If the sum of disease weights of the diagnostic factors in the personal examination data but not in the selected disease model is equal to or greater than the second threshold value, two or more diagnostic factors being the first and second most related with the target disease may be determined as diagnostic factors of the new disease model.

In response to the determination of the diagnostic factors for the new disease model, the new disease model is created (650) to include the determined diagnostic factors. The number of patients having the target disease including the determined diagnostic factors determined is computed by searching a patient DB. If the computed number of patients is equal to or greater than a third threshold value, the new disease model may be created to include the determined diagnostic factors.

If the computed number of patients is less than the third threshold value, the number of patients having the target disease including the determined diagnostic factors, while excluding the diagnostic factor being the least related with the target disease, may be computed.

In response to the creation of the new disease model, the created new disease model is registered (660) in a disease model DB. The patient DB may be searched for the patients who show the determined diagnostic factors. The patients found because of the search may be classified into a group of patients having the target disease and a group of patients not having the target disease. Further, the accuracy of the new disease model with respect to the group of patients not having the target disease may be evaluated. If the measured accuracy is equal to or greater than a fourth threshold value, the new disease model may be registered in the disease model DB.

The diagnostic factors that are included in the registered new disease model are determined (670) as a diagnostic factor set for disease diagnosis. If the comparison result of the first threshold value with the sum of disease weights of the diagnostic factors in the personal examination data but not in the selected disease model shows that the sum of disease weights is smaller than the first threshold value, a diagnostic factor set is determined (680) with the diagnostic factors that are included in both the personal examination data and the selected disease model. The determination of the diagnostic factors as a diagnostic factor set for disease diagnosis and the determination of the diagnostic factor set with the diagnostic factors that are included in both the personal examination data and the selected disease model are used as data to predict the occurrence of disease or assess the severity of ongoing disease.

According to the teachings above, the diagnostic factor set may be determined according to the disease weights of the diagnostic factors that are provided in the personal examination data but not included in the disease model, so that accurate and reliable disease diagnosis may be possible.

The units described herein may be implemented using hardware components and software components, such as microphones, amplifiers, band-pass filters, audio to digital convertors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors. As used herein, a processing device configured to implement a function A includes a processor programmed to run specific software. In addition, a processing device configured to implement a function A, a function B, and a function C may include configurations, such as, for example, a processor configured to implement both functions A, B, and C, a first processor configured to implement function A, and a second processor configured to implement functions B and C, a first processor to implement function A, a second processor configured to implement function B, and a third processor configured to implement function C, a first processor configured to implement function A, and a second processor configured to implement functions B and C, a first processor configured to implement functions A, B, C, and a second processor configured to implement functions A, B, and C, and so on.

The software may include a computer program, a piece of code, an instruction, or some combination thereof, for independently or collectively instructing or configuring the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. In particular, the software and data may be stored by one or more computer readable recording mediums. The computer readable recording medium may include any data storage device that can store data which can be thereafter read by a computer system or processing device. Examples of the computer readable recording medium include read-only memory (ROM), random-access memory (RAM), CD-ROMs, magnetic tapes, floppy disks, optical data storage devices. In addition, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein.

In addition, program instructions to perform a method described herein, or one or more operations thereof, may be recorded, stored, or fixed in one or more computer-readable storage media. The program instructions may be implemented by a computer. For example, the computer may cause a processor to execute the program instructions. The media may include, alone or in combination with the program instructions, data files, data structures, and the like. Examples of computer-readable storage media include magnetic media, such as hard disks, floppy disks, and magnetic tape; optical media such as CD ROM disks and DVDs; magnetooptical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable storage mediums. In addition, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein. In addition, the described unit to perform an operation or a method may be hardware, software, or some combination of hardware and software. For example, the unit may be a software package running on a computer or the computer on which that software is running.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A diagnostic factor set determination apparatus, comprising:
a personal examination data acquiring unit configured to acquire personal examination data comprising a plurality of diagnostic factors;
a disease model selecting unit configured to select a disease model that comprises one or more of the plurality of diagnostic factors; and
a diagnostic factor processing unit configured to determine a diagnostic factor set according to a sum of disease weights of a first group of the plurality of diagnostic factors that is not in the selected disease model.

2. The apparatus of claim 1, wherein the diagnostic factor processing unit comprises a diagnostic factor determination unit configured to determine diagnostic factors of a new disease model from the plurality of diagnostic factors if the sum is determined to be equal to or greater than a first threshold value.

3. The apparatus of claim 2, wherein the diagnostic factor processing unit further comprises a comparison unit configured to conduct a comparison of the first threshold value and the sum and provide a result of the comparison to the diagnostic factor determination unit to determine the diagnostic factors of the new disease model;
preferably, wherein, if the comparison result indicates that the sum is less than the first threshold value, the diagnostic factor determination unit determines diagnostic factors included in both the personal examination data and the selected disease model as the diagnostic factor set for disease diagnosis.

4. The apparatus of claim 2 or 3, wherein the diagnostic factor determination unit is further configured to include a second group of the plurality of diagnostic factors in the diagnostic factors of the new disease model, the second group being in the acquired personal examination data and the selected disease model;
preferably, wherein the diagnostic factor determination unit is further configured to identify correlated diagnostic factors between diagnostic factors of the first group and diagnostic factors of the selected disease model that are not in the acquired personal examination data, and to include the correlated diagnostic factors in the diagnostic factors of the new disease model.

5. The apparatus of claim 4, wherein, if the correlated diagnostic factors are not identified, the diagnostic factor determination unit calculates relations between a target disease and each of the diagnostic factors of the first group, and includes a third group of diagnostic factors of the first group in the diagnostic factors of the new disease model, the diagnostic factors of the third group respectively having calculated relations that are greater than the calculated relations of any other of the diagnostic factors of the first group;
preferably, wherein, if the sum is determined to be less than a second threshold value, the diagnostic factor determining unit determines one diagnostic factor of the diagnostic factors of the first group having one of the calculated relations that is greater than the calculated relations of any other of the diagnostic factors of the first group, and includes the determined one diagnostic factor in the diagnostic factors of the new disease model, and if the sum is determined to be equal to or greater than the second threshold value, the diagnostic factor determination unit determines two or more diagnostic factors of the diagnostic factors of the first group respectively having calculated relations that are greater than the calculated relations of any other of the diagnostic factors of the first group of the plurality of diagnostic factors, and includes the determined two or more diagnostic factors in the diagnostic factors of the new disease model.

6. The apparatus of claim 2 or one of claims 3 to 5 in combination with claim 2, wherein the diagnostic factor processing unit further comprises a disease model creation unit configured to create the new disease model to include the determined diagnostic factors;
preferably, wherein the diagnostic factor determination unit is further configured to classify the determined diagnostic factors as the diagnostic factor set.

7. The apparatus of claim 6, wherein the disease model creation unit is further configured to compute a first plurality of patients having a disease including the determined diagnostic factors by searching a patient database, DB,
wherein, if the computed first plurality of patients is equal to or greater than a third threshold value, the disease model creation unit creates the new disease model to include the determined diagnostic factors, and
wherein, if the computed first plurality of patients is less than the third threshold value, the disease model creation unit computes a second plurality of patients having a target disease that includes the determined diagnostic factors except for one of the determined diagnostic factors having a relation with the target disease that is less than relations of any other of the determined diagnostic factors with the target disease.

8. The apparatus of claim 6 or 7, wherein the diagnostic factor processing unit further comprises a disease model registration unit configured to register the created new disease model in a disease model database (DB).

9. The apparatus of claim 8, wherein the disease model DB stores disease models for a plurality of diseases, the plurality of diseases comprising the target disease,
wherein the disease model selecting unit is further configured to find disease models corresponding to the target disease by searching the disease model DB, and
wherein the disease model selecting unit is further configured to select one of the disease models corresponding to the target disease that comprises the one or more of the plurality of diagnostic factors.

10. The apparatus of claim 8 or 9, wherein the disease model registration unit is further configured to search a patient model DB for patients having a target disease comprising the determined diagnostic factors,
wherein the disease model registration unit is further configured to classify patients found from the search of the patient model DB into a group of patients having the target disease and a group of patients not having the target disease, and
wherein, if diagnosis accuracy is measured with respect to the group of patients not having the target disease and the diagnosis accuracy is equal to or greater than a fourth threshold value, the disease model registration unit registers the created new disease model in the disease model DB.

11. The apparatus of one of claims 1 to 10, wherein the disease model selecting unit is further configured to select the disease model from a plurality of disease models comprising one or more of the plurality of diagnostic factors, the selected disease model having a diagnosis accuracy that is greater than diagnosis accuracies of any other disease model of the plurality of disease models.

12. The apparatus of one of claims 1 to 11, wherein the selected disease model is one of a plurality of disease models, and
wherein the selected disease model comprises a greater number of the plurality of diagnostic factors than any other disease model of the plurality of disease models.

13. A diagnostic factor set determination method, comprising:
acquiring personal examination data comprising a plurality of diagnostic factors;
selecting a disease model that comprises one or more of the plurality of diagnostic factors of the acquired personal examination data; and
determining a diagnostic factor set according to a sum of disease weights of a first group of the plurality of diagnostic factors that is not in the selected disease model.

14. The method of claim 13, further comprising:
conducting a comparison of the first threshold value and the sum; and
if the comparison determines that the sum is equal to or greater than the first threshold value, determining diagnostic factors of a new disease model from the plurality of diagnostic factors.

15. The method of claim 14, further comprising:
creating the new disease model to include the determined diagnostic factors;
registering the created new disease model in a disease model database, DB; and
classifying the determined diagnostic factors as the diagnostic factor set.
